# EUROPEAN PATENT APPLICATION

(11) **EP 4 023 148 A1**
(43) Date of publication of application: **06.07.2022**
(21) Application number: 20858638.8
(22) Date of filing: 04.08.2020
(51) Int. Cl.: A61B 5/02, A61B 5/145

(54) **ELECTRONIC DEVICE**

(30) Priority: 27.08.2019 JP 2019155020
(71) Applicant: Kyocera Corporation, Kyoto-shi, Kyoto 612-8501 (JP)
(72) Inventor: AJIMA Hiromi, Kyoto-shi, Kyoto 612-8501 (JP)
(74) Representative: Viering, Jentschura & Partner mbB Patent- und Rechtsanwälte
(86) International application number: PCT/JP2020/029907
(87) International publication number: WO 2021/039318

(57) **Abstract**

An electronic device includes a sensor capable of detecting pulsation at a target portion of a subject; a housing, at least a part of which includes the sensor; a stand part that supports the housing, and that leans against the target portion via the housing; and an elastic member disposed between the housing and the stand part.

## Description

### CROSS REFERENCE TO RELATED APPLICATION

This application claims priority to and the benefit of Japanese Patent Application No. 2019-155020 filed on August 27, 2019, the entire contents of which are incorporated herein by reference.

### TECHNICAL FIELD

The present disclosure relates to an electronic device.

### BACKGROUND

Conventionally, there has been known an electronic device for measuring biological information at a target portion, such as a wrist, of a subject. For example, PTL 1 discloses an electronic device that counts pulse beats of a subject by being attached to a wrist of the subject.

### CITATION LIST

### Patent Literature

PTL 1: Japanese Patent Laid-Open No. 2002-360530

### SUMMARY

An electronic device according to an aspect includes a sensor capable of detecting pulsation at a target portion of a subject; a housing, at least a part of which includes the sensor; a stand part that supports the housing, and that leans against the target portion via the housing; and an elastic member disposed between the housing and the stand part.

### BRIEF DESCRIPTION OF THE DRAWINGS

In the accompanying drawings:
FIG. 1 is a diagram illustrating a mode of use of an electronic device according to an embodiment;
FIG. 2 is a diagram for describing a target portion of a subject;
FIG. 3 is a diagram illustrating an appearance of the electronic device according to an embodiment;
FIG. 4 is a diagram illustrating the appearance of the electronic device according to an embodiment;
FIG. 5 is a diagram illustrating the appearance of the electronic device according to an embodiment;
FIG. 6 is a diagram illustrating the appearance of the electronic device according to an embodiment;
FIG. 7 is a diagram illustrating the electronic device according to an embodiment and a wrist of a subject;
FIG. 8 is a diagram illustrating a cross-section of the electronic device according to an embodiment;
FIG. 9 is a diagram illustrating the cross-section of the electronic device according to an embodiment;
FIG. 10 is a diagram illustrating a mode of use of the electronic device according to an embodiment;
FIG. 11 is a functional block diagram illustrating a schematic configuration of the electronic device according to an embodiment;
FIG. 12 is a diagram illustrating an example of a pulse wave that is acquired by a sensor unit;
FIG. 13 is a diagram illustrating a change over time in a calculated AI;
FIG. 14 is a diagram illustrating the calculated AI and a measurement result of a blood glucose level;
FIG. 15 is a diagram illustrating a relationship between the calculated AI and the blood glucose level;
FIG. 16 is a diagram illustrating a calculated AI and a measurement result of a neutral lipid level;
FIG. 17 is a flow illustrating a procedure for estimating fluidity of blood and states of glucose metabolism and lipid metabolism; and
FIG. 18 is a schematic diagram illustrating a schematic configuration of a system according to an embodiment.

### DETAILED DESCRIPTION

Convenience of an electronic device is enhanced if biological information of a subject can be measured with ease. An object of the present disclosure is to provide a highly convenient electronic device. According to the present disclosure, an electronic device with enhanced convenience may be provided. In the following, an embodiment will be described in detail with reference to the drawings.

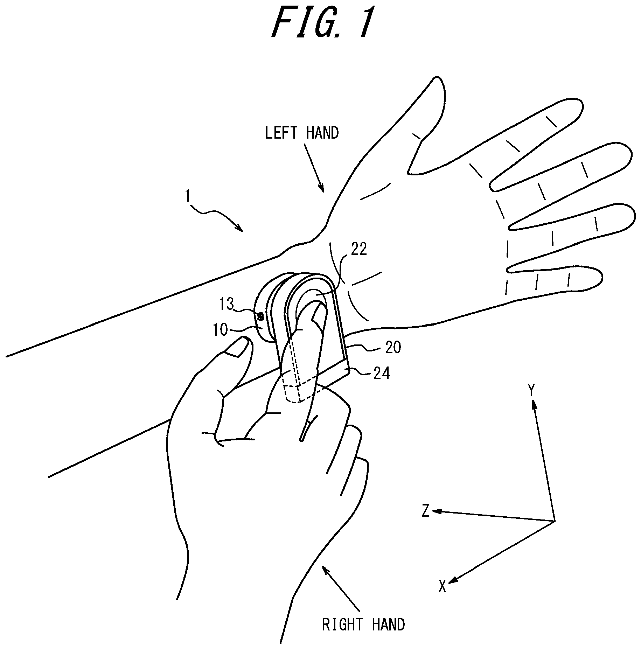

FIG. 1 is a diagram for describing a mode of use of an electronic device according to an embodiment. That is, FIG. 1 is a diagram illustrating a subject measuring biological information with the electronic device according to an embodiment.

As illustrated in FIG. 1, an electronic device 1 according to an embodiment is capable of measuring biological information of a subject using a part of the subject, such as a wrist, as a target portion. In the example illustrated in FIG. 1, the electronic device 1 is in contact with a target portion on a left wrist of the subject. In the example illustrated in FIG. 1, a wrist of the subject between a palm of a left hand and an elbow is taken as a target portion, and the electronic device 1 is in contact with the target portion.

As illustrated in FIG. 1, the electronic device 1 according to an embodiment includes a housing 10 and a stand part 20. The housing 10 may include a switch 13 for switching between on and off of the electronic device 1, and the like. As described later, the housing 10 includes a sensor 50 that is capable of detecting pulsation at a target portion of the subject. Moreover, the stand part 20 may include a press part 22 that is a part to be pressed by the subject or the like. Furthermore, the stand part 20 may include an extendable part 24 that can be extended. Each functional part of the electronic device 1 will be described later in greater detail.

A positive direction of a Y-axis illustrated in FIG. 1 will alternatively be referred to as an "up" direction as appropriate. Furthermore, a negative direction of the Y-axis illustrated in FIG. 1 will alternatively be referred to as a "down" direction as appropriate. That is, the up direction and the down direction illustrated in FIG. 1 may be approximately the same as an up direction and a down direction as seen from the subject.

In FIG. 1, a part of the electronic device 1 that is seen from a viewpoint facing a positive direction of a Z-axis will be referred to as a "rear side" of the electronic device 1. That is, in FIG. 1, the rear side of the electronic device 1 is a part of the stand part 20 of the electronic device 1 where the press part 22 is seen in plan view. Furthermore, in FIG. 1, a part of the electronic device 1 that is seen from a viewpoint facing a negative direction of the Z-axis will be referred to as a "front side" of the electronic device 1. That is, in FIG. 1, the front side of the electronic device 1 is a part of the housing 10 of the electronic device 1 where a surface that is to contact the target portion of the subject is seen in plan view.

A preparation for measuring biological information of a subject using the electronic device 1 as illustrated in FIG. 1 may be as follows, for example. First, the subject may place an arm (a left arm of the subject in the example illustrated in FIG. 1) where the biological information is to be measured on a stable supporting structure such as a table or a desk, for example. In FIG. 1, a supporting structure such as a table or a desk mentioned above may include a top board (a table top) that is parallel to an XZ plane illustrated in the drawing (that is, perpendicular to the Y-axis), for example. That is, the subject may place the arm where the biological information is to be measured on a supporting structure that includes a table top that is perpendicular to the Y-axis illustrated in the drawing. At this time, a palm of a hand (a left hand illustrated in FIG. 1) of the subject for measuring the biological information may face in the negative direction of the Z-axis illustrated in the drawing, or may face somewhat in the positive direction of the Y-axis from the negative direction of the Z-axis.

Next, the subject may bring the housing 10 of the electronic device 1 into contact with the target portion of the subject by causing the stand part 20 of the electronic device 1 to rest on the target portion. At the time of placement of the stand part 20 of the electronic device 1, the extendable part 24 of the stand part 20 may be made to stand on the top board (the table top) of a supporting structure such as a table or a desk mentioned above, for example. At this time, the subject may bring the housing 10 of the electronic device 1 into contact with the target portion in such a way that the sensor 50 of the electronic device 1 is positioned at a position where pulsation at the target portion can be desirably detected. In this case, the subject may perform positioning of the electronic device 1 by using a hand not used for measurement of the biological information (that is, a right hand of the subject in the example illustrated in FIG. 1).

Next, as illustrated in FIG. 1, the subject may press the electronic device 1 onto the target portion with a finger of the hand not used for measurement of the biological information (that is, the right hand of the subject in the example illustrated in FIG. 1), for example. In the example illustrated in FIG. 1, the electronic device 1 is pressed onto the target portion with an index finger of the right hand of the subject. As illustrated in FIG. 1, the stand part 20 of the electronic device 1 according to an embodiment may include the press part 22 that is to be pressed by the subject or the like. As illustrated in FIG. 1, the electronic device 1 according to an embodiment measures the biological information of the subject while being pressed onto the target portion. The finger with which the subject presses the electronic device 1 onto the target portion is not limited to the index finger of the right hand. The electronic device 1 may be pressed in any manner as long as the electronic device 1 may be pressed onto the target portion with an appropriate pressure.

The electronic device 1 may detect pulsation at the target portion of the subject by being placed in contact with the target portion. The target portion of the subject here may be a part where an ulnar artery or a radial artery of the subject is present under the skin, for example. Furthermore, the target portion of the subject is not limited to a part where the ulnar artery or the radial artery of the subject is present under the skin, and may be any part where pulsation of the subject can be detected. In FIG. 1, a part where the radial artery is present under the skin at the wrist of the subject is taken as the target portion, and a state where the electronic device 1 is in contact with the target portion is illustrated.

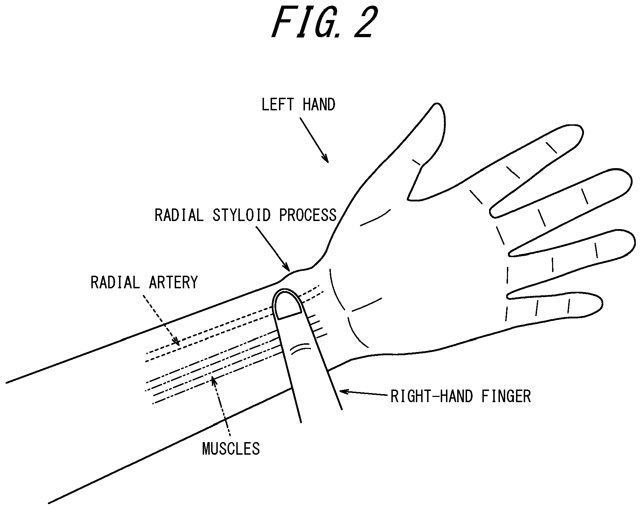

FIG. 2 is a diagram for describing the target portion of the subject. More specifically, FIG. 2 illustrates an example of a state where the subject is searching for a part, at the target portion of his/hers, where pulsation can be desirably detected, before measuring the biological information with the electronic device 1. That is, FIG. 2 illustrates a state where the subject is searching for a part, at the target portion on his/her left hand, where pulsation can be desirably detected, with a finger of his/her right hand. In FIG. 2, as in FIG. 1, the subject may be assumed to be placing his/her left arm on a supporting structure such as a table or a desk. Moreover, in FIG. 2, the radial artery and muscles present under the skin of the arm of the subject are illustrated by dashed lines and dash-dot-dotted lines.

As described above, the subject may bring the housing 10 of the electronic device 1 into contact with the target portion in such a way that the sensor 50 of the electronic device 1 is positioned at a position where pulsation can be desirably detected. A position, at the target portion of the subject, where pulsation can be desirably detected is different for each individual (each person). Accordingly, the subject may search for a position, at the target portion of his/hers, where pulsation can be desirably detected, before measuring the biological information with the electronic device 1.

In many cases, a position where pulsation can be desirably detected near the wrist of a subject is a position where there is the radial artery under the skin, and is, more particularly, a position where a radial styloid process is present under the skin or a periphery thereof. At a part where there is the radial artery above the radial styloid process, the radial artery is placed above the radial styloid process, which is relatively hard. At such a position, a contracting movement of the radial artery due to pulsation is transmitted to the skin of the subject, which is relatively soft, more easily than to a side of the radial styloid process, which is relatively hard. Accordingly, such a position may be taken as the target portion at the time of measuring the biological information of the subject with the electronic device 1 according to an embodiment.

As illustrated in FIG. 2, it is assumed that the subject found, with a fingertip of his/her right hand, desirable pulsation at a position, as illustrated in the drawing, near the wrist of his/her left hand, for example. In this case, the subject may take the position where desirable pulsation is found with the fingertip of his/her right hand as the target portion. The subject may thus bring the housing 10 of the electronic device 1 into contact with the target portion, as illustrated in FIG. 1. Moreover, if the position of the muscles illustrated in FIG. 2 is greatly included in the target portion, pulsation of the radial artery is possibly not desirably transmitted to the housing 10 (and the sensor 50) of the electronic device 1. Accordingly, at the time of bringing the housing 10 of the electronic device 1 into contact with the target portion, the subject may press the housing 10 (and the sensor 50) of the electronic device 1 onto the radial artery while avoiding the muscles as much as possible. A part of the housing 10 of the electronic device 1 that is to be brought into contact with the target portion of the subject will be described later in greater detail. Moreover, as illustrated in FIG. 1, at the time of measurement of the biological information of the subject with the electronic device 1, the subject may try to be in a psychological state where the entire body is relaxed, and may slightly open the palm of the hand for measuring the biological information (for example, the left hand).

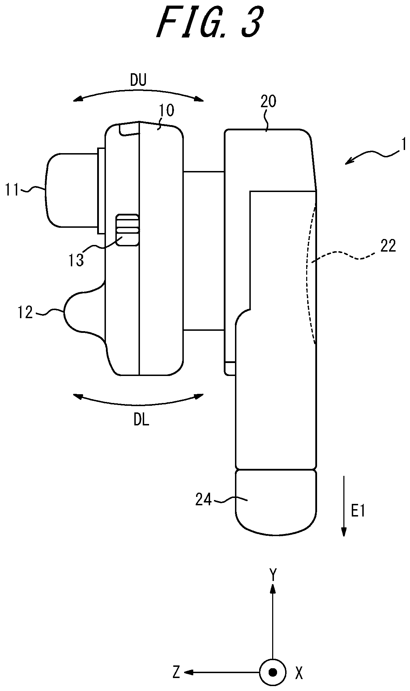

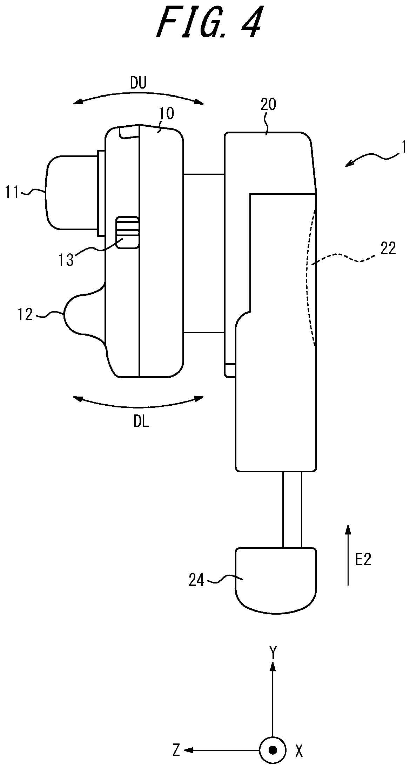

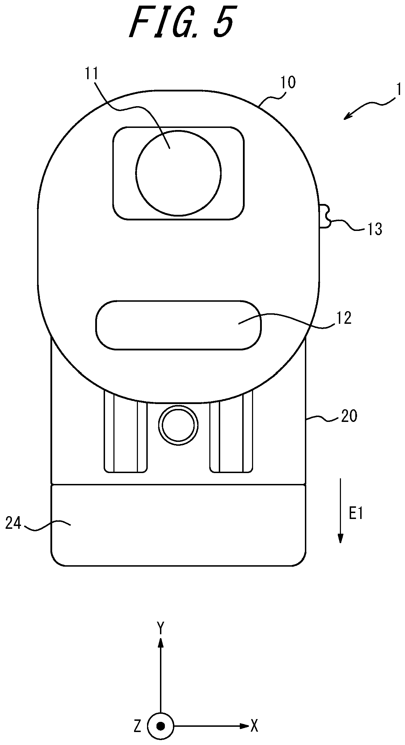

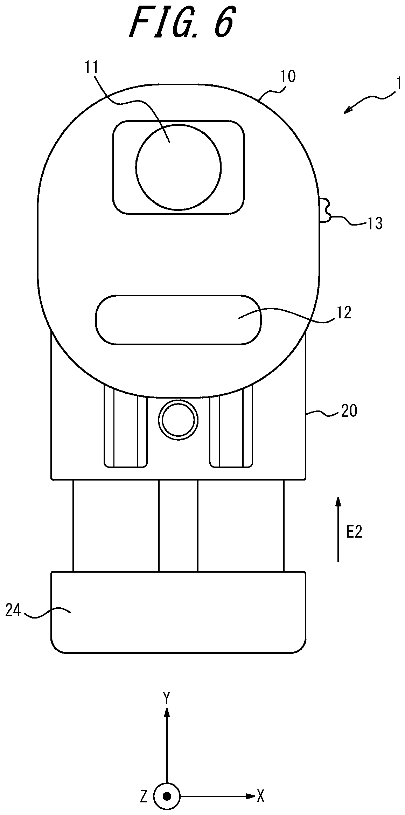

Next, a structure of the electronic device 1 according to an embodiment will be further described. FIGS. 3 and 4 are diagrams illustrating a state where the electronic device 1 as illustrated in FIG. 1 is seen from a viewpoint facing a negative direction of an X-axis. That is, FIGS. 3 and 4 are diagrams illustrating a right side surface of the electronic device 1 as illustrated in FIG. 1. Furthermore, FIGS. 5 and 6 are diagrams illustrating a state where the electronic device 1 as illustrated in FIG. 1 is seen from a viewpoint facing the negative direction of the Z-axis. That is, FIGS. 5 and 6 are diagrams illustrating the front side of the electronic device 1 as illustrated in FIG. 1.

As illustrated in FIGS. 3 to 6, the electronic device 1 includes the housing 10 and the stand part 20. The housing 10 and the stand part 20 of the electronic device 1 are connected by an elastic member, as described later. For example, the housing 10 and/or the stand part 20 may be formed of a material such as ceramics, iron or other metal, resin, plastic, or aluminum. The housing 10 and/or the stand part 20 may be formed of a material that is hard and light. The material of the housing 10 and/or the stand part 20 is not particularly limited, but may have strength that is sufficient to function as a measurement device. Furthermore, the material of the housing 10 and/or the stand part 20 is not excessively heavy and may be relatively light.

A size of the housing 10 and/or the stand part 20 of the electronic device 1 is not particularly limited, but the housing 10 and/or the stand part 20 may be made relatively small taking into account the convenience at the time of carrying and/or the ease of measurement, for example. The electronic device 1 as a whole may have a size that can be contained in a cube or a cuboid having sides that are around 7 cm, for example. However, in an embodiment, the size of the electronic device 1 as a whole may be larger or smaller than the size described above. Furthermore, a shape of each part of the electronic device 1, such as the housing 10 and the stand part 20, is not limited to the shape as illustrated in the drawings, and various shapes may be used taking into account functionality and/or design as a measurement device, for example.

As described later, the housing 10 and the stand part 20 may be moved relative to each other with a certain level of freedom. That is, with the electronic device 1, even in a state where the housing 10 is fixed, the stand part 20 may be moved with a certain level of freedom. Furthermore, with the electronic device 1, even in a state where the stand part 20 is fixed, the housing 10 may be moved with a certain level of freedom. For example, as illustrated in FIGS. 3 and 4, with the electronic device 1, the housing 10 may be moved with a certain level of freedom in directions indicated by an arrow DU and/or an arrow DL in the drawings.

As illustrated in FIGS. 3 to 6, the stand part 20 of the electronic device 1 may include the extendable part 24 at its lower end, for example. The extendable part 24 is extendable from the stand part 20. FIGS. 3 and 5 illustrate a state where the extendable part 24 is not extended from the stand part 20. FIGS. 4 and 6 illustrate a state where the extendable part 24 is extended from the stand part 20. That is, when the extendable part 24 is extended in a direction of an arrow E1 in FIGS. 3 and 5, the extendable part 24 may be extended to stretch from the stand part 20, as illustrated in FIGS. 4 and 6. When the extendable part 24 contracts in a direction of an arrow E2 in FIGS. 4 and 6, the extendable part 24 may be returned to its original position, as illustrated in FIGS. 3 and 5. In this manner, with the electronic device 1 according to an embodiment, a length of the stand part 20 in an up-down direction may be adjusted by causing the extendable part 24 to extend or contract.

Furthermore, by making the length of the stand part 20 in the up-down direction adjustable by the extendable part 24, a position of the housing 10 in the up-down direction (a height direction) is made adjustable. Accordingly, even when a diameter of the left wrist of a subject as illustrated in FIG. 1 is different for each individual to a certain extent, the position at which the housing 10 contacts the target portion of a subject may be adjusted according to the position of the target portion of the subject in the up-down direction. In this manner, with the electronic device 1 according to an embodiment, the stand part 20 is capable of extend or contract in a predetermined direction as indicated by the arrow E1 and/or the arrow E2, so that the position of the housing 10 in the height direction may be made adjustable by enabling.

The extendable part 24 may be extendable from the stand part 20 in a stepless manner. That is, the extendable part 24 may be allowed to be positioned at any position up to a predetermined length, for example. According to such a structure, even when a diameter of a wrist including the target portion of a subject is different for each individual, the position at which the housing 10 of the electronic device 1 contacts the target portion of a subject may be finely adjusted.

Alternatively, the extendable part 24 may be extendable from the stand part 20 in a stepwise manner. That is, the extendable part 24 may include a mechanism that allows positioning at a plurality of predetermined positions set in advance, up to a predetermined length, for example. The extendable part 24 may include a mechanism such as a multistage stay that is locked in multiple stages at the time of extension from the stand part 20, for example. According to such a structure, at the time of a subject measuring the biological information with the electronic device 1, a same measurement environment as at the time of previous measurement may be easily reproduced, for example. In this manner, with the electronic device 1 according to an embodiment, the stand part 20 may be capable of extending or contracting in a stepwise manner in a predetermined direction as indicated by the arrow E1 and/or the arrow E2 by including the extendable part 24, for example.

As illustrated in FIGS. 3 to 6, the housing 10 of the electronic device 1 may include a first contact part 11 as a part that is to contact the target portion of the subject. The first contact part 11 may be provided on a target portion side of the housing 10. For example, the first contact part 11 may function as a member such as a pulse pad. Furthermore, as illustrated in FIGS. 3 to 6, the housing 10 of the electronic device 1 may include a second contact part 12 as a part that is to contact the target portion of the subject or a periphery of the target portion. The second contact part 12 may contact the target portion of the subject, near a position where the first contact part 11 contacts. The second contact part 12 may also be provided on the target portion side of the housing 10 (on the side of the wrist of the subject).

As described above, the first contact part 11 is a member that is to be appropriately brought into contact with the target portion of the subject at the time of measurement of the biological information of the subject with the electronic device 1. Accordingly, the first contact part 11 may have a size that allows the first contact part 11 to appropriately contact the subject at a part where the ulnar artery or the radial artery is present under the skin, for example. As illustrated in FIGS. 5 and 6, the first contact part 11 may have a width of about 1 cm to 1.5 cm in an X-axis direction or a Y-axis direction, for example. Alternatively, the width of the first contact part 11 in the X-axis direction or the Y-axis direction may be other than between around 1 cm and 1.5 cm.

For example, the first contact part 11 and the second contact part 12 may be formed of a material such as ceramics, iron or other metal, resin, plastic, or aluminum. The first contact part 11 and the second contact part 12 may be formed of a material that is hard and light. The material of the first contact part 11 and the second contact part 12 is not particularly limited. Like the housing 10 and/or the stand part 20, the material of the first contact part 11 and the second contact part 12 may be relatively light, with strength that is sufficient to function as a measurement device.

Furthermore, as illustrated in FIGS. 3 to 6, the housing 10 of the electronic device 1 may include the switch 13. The switch 13 may be a switch that switches between on and off of the electronic device 1, for example. Furthermore, the switch 13 may be a switch for causing the electronic device 1 to start measuring the biological information, for example. FIGS. 3 to 6 illustrate an example where the switch 13 is formed as a slide switch. However, the switch 13 may be any switch such as a press button switch, for example. In the case where the switch 13 is a press button switch, the number of times the switch 13 is pressed and/or a duration when the switch 13 is pressed may correspond to a respective operation of the electronic device 1, for example. The switch 13 may be disposed at any position without being limited to the example illustrated in FIGS. 3 to 6. For example, the switch 13 may be disposed on the stand part 20.

Next, a mode of measurement of the biological information by the electronic device 1 according to an embodiment will be described.

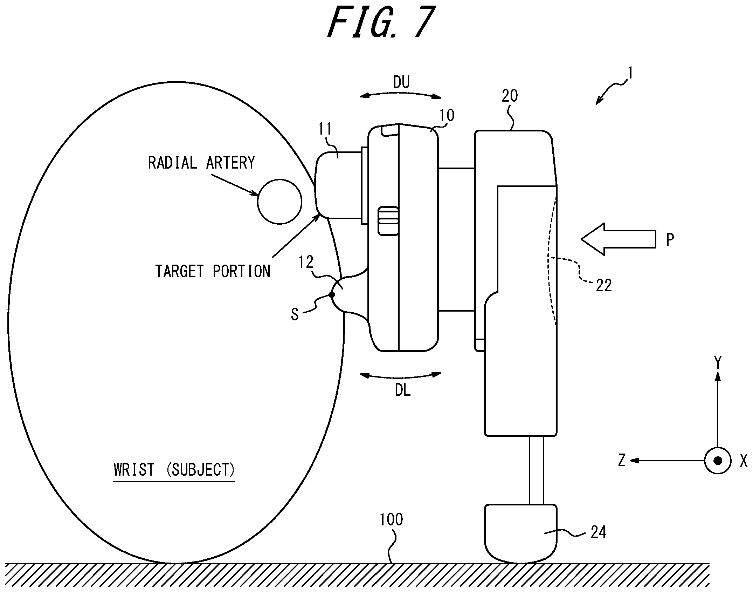

FIG. 7 illustrates a subject measuring the biological information with the electronic device 1. FIG. 7 is a diagram illustrating a state where the electronic device 1 illustrated in FIG. 1 is seen from a side, together with a cross-section of a wrist of the subject. That is, FIG. 7 is a diagram illustrating a state where the electronic device 1 as illustrated in FIG. 1 is seen from a viewpoint facing the negative direction of the X-axis, together with a cross-section of a wrist of the subject.

As illustrated in FIG. 7, a left wrist of the subject is placed on an upper surface of a top board (a table top) 100 of a supporting structure such as a table or a desk. Furthermore, as illustrated in FIG. 7, the electronic device 1 is leaning against the target portion of the subject with the extendable part 24 at the lower end of the stand part 20 in contact with the upper surface of the top board (the table top) 100 of a supporting structure such as table or a desk. The example illustrated in FIG. 7 illustrates a state where the extendable part 24 of the stand part 20 of the electronic device 1 is somewhat extended. In such an arrangement state, the electronic device 1 may start measurement of the biological information by being pressed in a direction of an arrow P illustrated in FIG. 7 with a right hand of the subject, for example. The electronic device 1 of the present disclosure may alternatively be used with the extendable part 24 at the lower end of the stand part 20 not in contact with the upper surface of the top board (the table top) 100 of a supporting structure such as table or a desk.

As illustrated in FIG. 7, the first contact part 11 may directly or indirectly contact the target portion of the subject. Furthermore, as illustrated in FIG. 7, the second contact part 12 may directly or indirectly contact a periphery of the part where the first contact part 11 is in contact with the target portion of the subject. A surface including the target portion on the wrist of a subject generally has a curved shape as illustrated in FIG. 7. Accordingly, if the first contact part 11 and the second contact part 12 of the housing 10 have a same length in the Z-axis direction, the first contact part 11 possibly floats above the wrist of the subject (that is, the target portion) in a state where the second contact part 12 is in contact with the wrist of the subject. Accordingly, as illustrated in FIG. 7, in an embodiment, the length of the first contact part 11 in the Z-axis direction may be made longer than the length of the second contact part in the Z-axis direction. This allows the first contact part 11 to be appropriately in contact with the target portion of the subject in a state where the second contact part 12 is in contact with a part of the wrist of the subject (for example, a part indicated by S in FIG. 7).

In this manner, in an embodiment, the first contact part 11 may protrude farther from the housing 10 in the Z-axis direction as illustrated in FIG. 7 than the second contact part 12 does, for example. That is, a length by which the first contact part 11 protrudes from the housing 10 in the positive direction of the Z-axis may be greater than a length by which the second contact part 12 protrudes from the housing 10 in the positive direction of the Z-axis.

A shape of the first contact part 11 may be any shape that can appropriately contact the target portion of the subject, without being limited to the shape as illustrated in FIGS. 3 to 7. Similarly, a shape of the second contact part 12 may be any shape that can appropriately contact a part of the wrist of the subject (for example, the part indicated by S in FIG. 7), without being limited to the shape as illustrated in FIGS. 3 to 7.

As illustrated in FIG. 7, the stand part 20 of the electronic device 1 may include the press part 22. The press part 22 may be a part of the electronic device 1 that is pressed with a fingertip or the like of the subject. That is, the subject or the like may perceive that the press part 22 is to be pressed with a fingertip or the like, by looking at (or touching) the press part 22. As illustrated in FIG. 7, the press part 22 may be formed on a rear side of the stand part 20 (that is, a surface facing the negative direction of the Z-axis). In the example illustrated in FIG. 7, the press part 22 is formed at a position that is slightly in the up direction (the positive direction of the Y-axis) from a center of the stand part 20. However, the press part 22 may be formed at various positions, such as approximately at the center of the stand part 20, depending on the mode of measurement of the biological information with the electronic device 1.

Furthermore, in the example illustrated in FIG. 7, the press part 22 is illustrated as a shallow recessed part formed in the stand part 20. However, a shape of the press part 22 is not limited to a shallow recessed shape. For example, the press part 22 may be formed as a low protruding part that is formed on the stand part 20. Furthermore, the press part 22 may simply be a mark that is painted on the stand part 20 with a paint or the like. The press part 22 may take any form as long as a part, of the electronic device 1, that is to be pressed with a fingertip or the like of the subject may be indicated.

When the first contact part 11 is in contact with the target portion, such as the wrist of the subject, and the press part 22 is pressed with a fingertip or the like of the subject, the electronic device 1 is placed in a state for measuring the biological information, as illustrated in FIG. 1 or 7. At the time of bringing the electronic device 1 into contact with the target portion, such as the wrist of the subject, the first contact part 11 may be positioned to contact the target portion of the subject. At this time, as illustrated in FIG. 7, the first contact part 11 may be positioned so as to contact a part where the ulnar artery or the radial artery of the subject is present under the skin, for example. That is, the target portion at which the electronic device 1 according to an embodiment measures the biological information of the subject may be a position where the radial artery or the ulnar artery of the subject runs below the skin, for example.

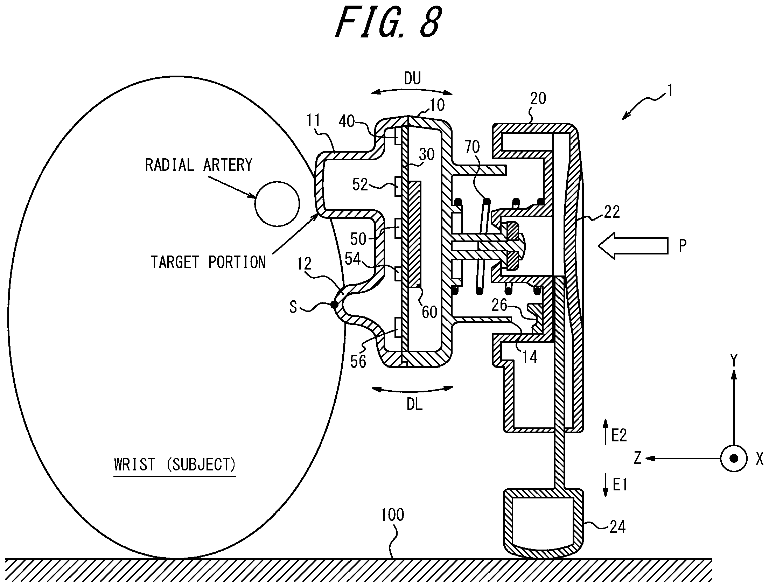

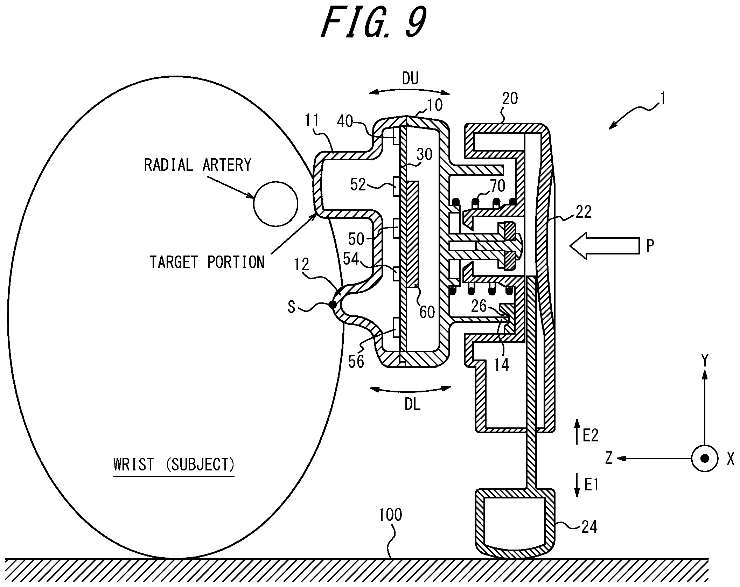

FIGS. 8 and 9 are diagrams illustrating a cross-section of the electronic device 1 together with a cross-section of a wrist of a subject. FIG. 8 is a diagram illustrating a cross-section of the electronic device 1 illustrated in FIG. 7, together with a cross-section of a wrist of a subject. FIG. 9 is a cross-sectional diagram illustrating a state where the press part 22 of the electronic device 1 illustrated in FIG. 8 is pressed in the direction of the arrow P illustrated in the drawing.

As illustrated in FIGS. 8 and 9, externally, the electronic device 1 includes the housing 10 and the stand part 20. Furthermore, as described above, the housing 10 includes the first contact part 11 and the second contact part 12. Moreover, the stand part 20 includes the press part 22 and the extendable part 24.

Moreover, as illustrated in FIGS. 8 and 9, the housing 10 of the electronic device 1 may further include a substrate 30. The substrate 30 may be a general circuit board where various electronic components and the like may be mounted. In an embodiment, the substrate 30 may be built inside the housing 10 of the electronic device 1.

Various electronic components may be mounted on the substrate 30, on surfaces facing the positive directions of the Z-axis. In the example illustrated in FIGS. 8 and 9, a notification interface 40, the sensor 50, a controller 52, a storage 54, and a communication interface 56 are mounted on the substrate 30, on the surfaces facing the positive directions of the Z-axis. Furthermore, the switch 13 as described above may also be mounted on the substrate 30.

The notification interface 40 notifies the subject or the like of information such as a measurement result of the biological information, for example. The notification interface 40 may be a light emitting unit that uses light-emitting diode (LED) or the like. Alternatively, the notification interface 40 may be a display device such as a liquid crystal display (LCD), an organic electro-luminescence display (OELD), an inorganic electro-luminescence display (IELD), or the like. When a display device as mentioned above is used as the notification interface 40, relatively detailed information, such as a state of glucose metabolism or lipid metabolism of the subject, may also be displayed, for example.

In addition to information such as a measurement result of the biological information, the notification interface 40 may also notify the subject of information about on/off of the electronic device 1, whether the biological information is being measured or not, and the like, for example. At this time, the notification interface 40 may notify of information about on/off of the electronic device 1 or whether the biological information is being measured or not, through light emission in a mode different from at the time of notifying of information such as a measurement result of the biological information, for example.

In an embodiment, the notification interface 40 does not have to be a light emitting unit. For example, the notification interface 40 may be an audio output unit, such as a speaker or a buzzer. In this case, the notification interface 40 may notify the subject or the like of information such as a measurement result of the biological information using various sounds or voices, for example.

Furthermore, in an embodiment, the notification interface 40 may be a tactile sensation presenting unit, such as a vibrator or a piezoelectric device, for example. In this case, the notification interface 40 may notify the subject or the like of information such as a measurement result of the biological information through various vibrations or tactile feedbacks.

The sensor 50 includes an angular velocity sensor, for example, and detects pulsation at the target portion and acquires a pulse wave. The sensor 50 may detect a displacement in the position of the first contact part 11 (a pulse pad) based on the pulse wave of the subject. Moreover, the sensor 50 may be a sensor such as an accelerometer or a gyro sensor, for example. Furthermore, the sensor 50 may be an angular velocity sensor. The sensor 50 will be described later in greater detail.

As illustrated in FIGS. 8 and 9, the sensor 50 is fixed on the substrate 30. Furthermore, the substrate 30 is fixed inside the housing 10. Furthermore, the first contact part 11 is fixed on an outer side of the housing 10. Accordingly, movement of the first contact part 11 is transmitted to the sensor 50 via the housing 10 and the substrate 30. Accordingly, the sensor 50 may detect the pulsation at the target portion of the subject via the first contact part 11, the housing 10, and the substrate 30.

In the example illustrated in FIGS. 8 and 9, the sensor 50 is disposed in a state of being built inside the housing 10. However, in an embodiment, the sensor 50 does not have to be entirely built inside the housing 10. In an embodiment, the sensor 50 may be included in at least a part of the housing 10. The sensor 50 may have any structure to which movement of at least one of the first contact part 11, the housing 10, and the substrate 30 is transmitted.

The controller 52 is a processor that controls and manages the entire electronic device 1 including each functional block of the electronic device 1. Furthermore, the controller 52 is a processor that calculates an index that is based on a pulse wave propagation phenomenon, from a pulse wave that is acquired. The controller 52 is a processor such as a central processing unit (CPU) that executes a program specifying a control procedure and a program for calculating the index that is based on the pulse wave propagation phenomenon, and such programs are stored in a storage medium such as the storage 54, for example. Furthermore, the controller 52 estimates the state of glucose metabolism, lipid metabolism or the like of the subject based on the calculated index. The controller 52 may also notify the notification interface 40 of data.

The storage 54 stores programs and data. The storage 54 may include a semiconductor storage medium, and an arbitrary non-transitory storage medium such as a magnetic storage medium. The storage 54 may include a plurality of types of storage media. The storage 54 may include a combination of a portable storage medium, such as a memory card, an optical disk, a magneto-optical disk or the like, and a reading device for the storage medium. The storage 54 may include a storage device that is used as a temporary storage area such as a random access memory (RAM). The storage 54 stores various pieces of information and/or programs for causing the electronic device 1 to operate, and also functions as a working memory. For example, the storage 54 may store a measurement result of a pulse wave that is acquired by the sensor 50.

The communication interface 56 transmits/receives various pieces of data by performing wired or wireless communication with an external device. For example, the communication interface 56 communicates with an external device that stores biological information of a subject to manage a health state, and transmits, to the external device, a measurement result of a pulse wave measured by the electronic device 1 and/or a health state estimated by the electronic device 1. For example, the communication interface 56 may be a communication module that is compatible with Bluetooth^{®} (Bluetooth is a registered trademark in Japan, other countries, or both), Wi-Fi, or the like.

As illustrated in FIGS. 8 and 9, a battery 60 may be mounted on a surface of the substrate 30, on the surface facing the negative direction of the Z-axis. In this case, a battery holder for fixing the battery 60 may be disposed on the substrate 30, on the surface facing the negative direction of the Z-axis. The battery 60 may be any power source, such as a button battery (a coin-type battery) such as CR2032, for example. The battery 60 may alternatively be a rechargeable battery, for example. The battery 60 may include a lithium-ion battery and a control circuit for charging and discharging the lithium-ion battery, for example. The battery 60 may supply power to each functional unit of the electronic device 1.

Arrangement of the notification interface 40, the sensor 50, the controller 52, the storage 54, the communication interface 56, and the battery 60 is not limited to the example illustrated in FIGS. 8 and 9. For example, the functional units described above may be disposed on any positions on the substrate 30. Furthermore, the functional units described above may be disposed on one of two surfaces of the substrate 30 as appropriate. Furthermore, in a case where the electronic device 1 is connected to the external device in a wired or wireless manner, at least one of functional units including the switch 13, the notification interface 40, the controller 52, the storage 54, the communication interface 56 and the like may be provided in the external device as appropriate, for example.

As illustrated in FIGS. 8 and 9, an end portion of the housing 10 of the electronic device 1 facing the negative direction of the Z-axis is connected to an end portion of the stand part 20 facing the positive direction of the Z-axis. As illustrated in FIGS. 8 and 9, the housing 10 includes, on a side facing the negative direction of the Z-axis, a connection portion that is to be connected to the stand part 20. Furthermore, as illustrated in FIGS. 8 and 9, the stand part 20 includes, on a side facing the positive direction of the Z-axis, an opening portion where the connection portion of the housing 10 is to be inserted. In the example illustrated in FIGS. 8 and 9, the connection portion of the housing 10 has a size that is smaller than that of the opening portion of the stand part 20, and the connection portion of the housing 10 is inserted in the opening portion of the stand part 20. However, in an embodiment, the housing 10 may alternatively include an opening portion, and the stand part 20 may include an insertion portion. In this case, the opening portion of the housing 10 may have a size that is larger than that of the insertion portion of the stand part 20, and the insertion portion of the stand part 20 may be inserted in the opening portion of the housing 10. In either case, the housing 10 and the stand part 20 may be allowed to move with a certain level of freedom without interfering with each other.

As illustrated in FIGS. 8 and 9, the housing 10 and the stand part 20 of the electronic device 1 are connected to each other by an elastic member 70. In the example illustrated in FIGS. 8 and 9, the housing 10 and the stand part 20 are directly connected by the elastic member 70. However, the elastic member 70 may indirectly connect the housing 10 and the stand part 20, for example. In an embodiment, for example, the elastic member 70 may connect an arbitrary member of the housing 10 and an arbitrary member of the stand part 20. The elastic member 70 may be an elastic member that is deformable along at least one axis among three mutually perpendicular axes (such as the X-axis, the Y-axis, the Z-axis). The elastic member 70 is a three-dimensionally deformable member.

FIGS. 8 and 9 illustrate an example where the elastic member 70 is a spring such as a compression spring. However, in an embodiment, the elastic member 70 may be any elastic body having appropriate elasticity, such as a spring, resin, a sponge, a silicon sheet or the like, or a combination thereof. For example, the elastic member 70 may be formed from a silicon sheet having a predetermined thickness and predetermined elasticity.

FIG. 8 illustrates a state where the press part 22 of the stand part 20 is not pressed in the direction of the arrow P by the subject or the like. That is, FIG. 8 illustrates a state where the stand part 20 is not pressed toward the target portion. FIG. 9 illustrates a state where the press part 22 of the stand part 20 is pressed in the direction of the arrow P by the subject or the like. That is, FIG. 9 illustrates a state where the stand part 20 is pressed toward the target portion. The elastic member 70 is deformed by such pressure, and thus, a length of the elastic member 70 in the Z-axis direction illustrated in FIG. 9 is shorter than the length of the elastic member 70 in the Z-axis direction illustrated in FIG. 8.

In the example illustrated in FIG. 8, the electronic device 1 includes a stopper mechanism so that the housing 10 and the stand part 20 are not displaced by a distance of a predetermined length or more. That is, the electronic device 1 illustrated in FIG. 8 includes a mechanism that prevents the housing 10 from becoming detached or falling off from the stand part 20 even in a state where pressing in the direction of the arrow P in the drawing is not performed. FIG. 8 illustrates a state where a distance between the housing 10 and the stand part 20 is fixed while maintaining resilience of the elastic member 70 to a certain extent. In such a state, the distance between the housing 10 and the stand part 20 is such that the housing 10 and the stand part 20 are not displaced by a distance of a greater length.

When a pressure is applied in the direction of the arrow P in the drawing in the state illustrated in FIG. 8, the elastic member 70 is deformed in a contracting direction, as illustrated in FIG. 9. In the state illustrated in FIG. 9, a protruding part 14 of the housing 10 reaches and comes in contact with a receiving part 26 of the stand part 20. When the pressure in the direction of the arrow P in the drawing is reduced from this state, a state is reached where the protruding part 14 of the housing 10 does not contact the receiving part 26 of the stand part 20, while the elastic member 70 remains somewhat contracted. In this state, the position of the housing 10 may be displaced with a certain level of freedom relative to the stand part 20 that is connected via the elastic member 70. Accordingly, the electronic device 1 may desirably detect pulsation at the target portion of the subject.

In FIGS. 8 and 9, the elastic member 70 is a spring, such as a compression spring. However, as described above, the elastic member 70 may be a silicon sheet having a predetermined thickness, for example. In this case, the housing 10 and the stand part 20 may be bonded to the elastic member 70 with an adhesive, a double-sided tape or the like. Here, the elastic member 70 may be bonded to other members in such a way that deformation of the elastic member 70 is not greatly affected. That is, the elastic member 70 may be allowed to be deformed as appropriate even when the elastic member 70 is bonded to other members.

As described above, the electronic device 1 according to an embodiment includes the housing 10, the stand part 20, the sensor 50, and the elastic member 70. The housing 10 includes the sensor 50 in at least a part thereof. The sensor 50 is capable of detecting pulsation at the target portion of a subject. The stand part 20 is structured to support the housing 10, and to lean against the target portion via the housing 10. The elastic member 70 is disposed between the housing 10 and the stand part 20.

As illustrated in FIGS. 8 and 9, in a state where the electronic device 1 is leaning against the target portion of a subject, the first contact part 11 of the housing 10 is in contact with the target portion of the subject, that is, with the skin above the radial artery of the subject. Furthermore, the press part 22 of the stand part 20 is pressed toward the target portion, or in other words, in the direction of the arrow P, with a finger of the right hand of the subject, for example. Furthermore, the sensor 50 (together with the housing 10 and the first contact part 11) is biased toward the target portion of the subject by elasticity of the elastic member 70 that is disposed between the stand part 20 that is pressed via the press part 22 and the housing 10 including the sensor 50. Furthermore, the first contact part 11 that is biased by the elasticity of the elastic member 70 is in contact with the skin above the radial artery of the subject. In this case, the position of the first contact part 11 is displaced according to movement of the radial artery of the subject, or in other words, pulsation. Accordingly, the position of the sensor 50 that moves together with the first contact part 11 is also displaced according to movement of the radial artery of the subject, or in other words, pulsation. For example, as illustrated in FIGS. 8 and 9, in a state where the press part 22 is pressed by the subject in the direction of the arrow P, the position may be displaced in a direction as indicated by the arrow DU or the arrow DL around an axis S. The axis S here may be at a part where the second contact part 12 of the housing 10 contacts the wrist of the subject. In this case, a position that is pressed in the direction of the arrow P (that is, the position of the press part 22) may be a position between the axis S and the first contact part 11 (the target portion) on an XY plane.

In the present embodiment, the sensor 50 that moves together with the first contact part 11 is coupled to the stand part 20 (the press part 20) via the elastic member 70. Accordingly, the sensor 50 has a movable range where the sensor is allowed to move with a certain level of freedom by flexibility of the elastic member 70. Furthermore, movement of the sensor 50 is not easily obstructed due to the flexibility of the elastic member 70. Moreover, due to appropriate elasticity, the elastic member 70 deforms following pulsation at the target portion of the subject. Accordingly, the sensor 50 of the electronic device 1 according to the present embodiment may detect pulsation at the target portion of the subject with high sensitivity. Moreover, because the position of the electronic device 1 according to the present embodiment is displaced following the pulse wave, congestion in the subject can be eliminated and pain can be eliminated. In this manner, in the present embodiment, the elastic member 70 may be deformable in response to pulsation at the target portion of the subject. Furthermore, the elastic member 70 may be elastically deformable to the extent that allows the sensor 50 to detect pulsation at the target portion of the subject.

As described above, the electronic device 1 according to an embodiment may function as a small and light measurement device. The electronic device 1 according to an embodiment is superior in portability, and also enables the biological information of a subject to be measured with great ease. Furthermore, the electronic device 1 according to an embodiment is capable of independently measuring the biological information without coordinating with another external device or the like. In this case, there is no need to carry other accessories such as a cable. Accordingly, with the electronic device 1 according to an embodiment, convenience may be enhanced.

In an embodiment, the electronic device 1 may include a stopper-like mechanism between the housing 10 and the stand part 20. FIGS. 8 and 9 illustrate, as an example, a structure where the housing 10 includes the protruding part 14 and the stand part 20 includes the receiving part 26. That is, the housing 10 includes the protruding part 14 at a part of the connection portion to be connected to the stand part 20. Furthermore, the stand part 20 includes the receiving part 26 that is capable of receiving the protruding part 14, at a part of the opening portion where the connection portion of the housing 10 is to be inserted. In the following, the protruding part 14 and the receiving part 26 will be collectively referred to as "stopper (14, 26)".

As illustrated in FIGS. 8 and 9, the stopper (14, 26) is formed only at a part of the insertion portion of the housing 10 and the opening portion of the stand part 20 where the housing 10 and the stand part 20 are connected. For example, in the example illustrated in FIGS. 8 and 9, the stopper (14, 26) is formed only at a lower end of the part where the housing 10 and the stand part 20 are connected. The stopper (14, 26) is not formed at an upper end of the part where the housing 10 and the stand part 20 are connected. In an embodiment, the stopper (14, 26) does not have to be formed at the upper end of the part where the housing 10 and the stand part 20 are connected and also not at parts other than the lower end of the part where the housing 10 and the stand part 20 are connected.

By including the stopper (14, 26) only at a part as described above, movement of the housing 10 relative to the stand part 20 is not easily restricted even in a case where the subject or the like presses the stand part 20 (the press part 22) with a relatively strong force. For example, in the state illustrated in FIG. 8, the subject or the like is not pressing the stand part 20 (the press part 22) in the direction of the arrow P in the drawing with a strong force, and thus, the protruding part 14 and the receiving part 26 are not in contact with each other. In the state illustrated in FIG. 9, the subject or the like is pressing the stand part 20 (the press part 22) in the direction of the arrow P in the drawing with a strong force. Accordingly, the elastic member 70 is deformed and the position of the housing 10 is displaced relative to the stand part 20, and as a result, the protruding part 14 and the receiving part 26 are in contact with each other. Also in this case, the housing 10 and the stand part 20 are not in contact with each other at parts other than the part where the protruding part 14 and the receiving part 26 are in contact with each other. Accordingly, in relation to movement of the housing 10 relative to the stand part 20, movement as indicated by the arrow DL in the drawing may be somewhat restricted, but movement as indicated by the arrow DU in the drawing is hardly restricted. Accordingly, even in a case where the subject or the like presses the stand part 20 (the press part 22) with a relatively strong force, movement of the housing 10 relative to the stand part 20 is not easily restricted.

FIGS. 8 and 9 illustrate a structure where the housing 10 includes the protruding part 14 and the stand part 20 includes the receiving part 26, but a reversal thereof is also possible. That is, in an embodiment, the housing 10 may include the receiving part 26 and the stand part 20 may include the protruding part 14.

As described above, the electronic device 1 according to an embodiment may include the stopper (14, 26). The stopper (14, 26) may include the protruding part 14 and the receiving part 26. The protruding part 14 may be formed on one of the housing 10 and the stand part 20. The receiving part 26 may be formed on the other one of the housing 10 and the stand part 20. Furthermore, with the stopper (14, 26), the receiving part 26 may be capable of receiving the protruding part 14. Furthermore, in an embodiment, the stopper (14, 26) may be such that the housing 10 is allowed to partially come into contact with the stand part 20 when the position of the housing 10 is displaced relative to the stand part 20 by deformation of the elastic member 70.

In the present embodiment, the sensor 50 may be a sensor, such as a gyro sensor (a gyroscope), that detects, in relation to an object, at least one of an angle (an inclination), angular velocity, and angular acceleration about a plurality of axes, for example. In this case, the sensor 50 may detect complex movement based on the pulsation at the target portion of the subject in the form of parameters on the plurality of axes. Moreover, the sensor 50 may be a 6-axis sensor combining a 3-axis gyro sensor and a 3-axis accelerometer.

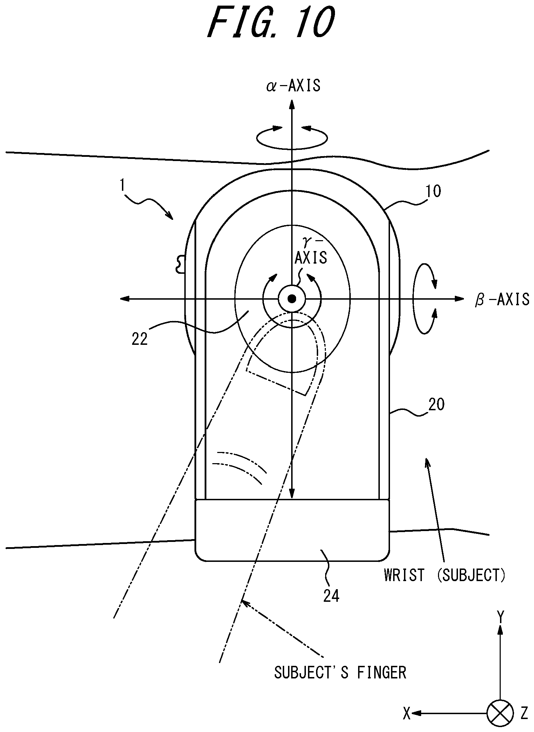

FIG. 10 is a diagram illustrating an example of a mode of use of the electronic device 1. FIG. 10 is a diagram illustrating the state illustrated in FIG. 1, in an enlarged manner and from another viewpoint.

For example, as illustrated in FIG. 10, the sensor 50 built inside the housing 10 of the electronic device 1 may detect rotational movement around each of three axes including an α-axis, a β-axis, and a γ-axis. The α-axis may be an axis that is along a direction that is approximately perpendicular to the radial artery of the subject, for example. Furthermore, the β-axis may be an axis that is along a direction that is approximately parallel to the radial artery of the subject, for example. Furthermore, the γ-axis may an axis that is along a direction that is approximately perpendicular to both the α-axis and the β-axis, for example.

In this manner, in the present embodiment, the sensor 50 may detect the pulsation at the target portion of the subject as a part of rotational movement around a predetermined axis. Furthermore, the sensor 50 may detect the pulsation at the target portion of the subject as rotational movement around at least two axes or as rotational movement around three axes. In the present disclosure, "rotational movement" does not necessarily have to be movement by which the position is displaced by at least one round of a circular path. For example, in the present disclosure, the rotational movement may be partial displacement of a position by less than one round of a circular path (such as displacement of a position along an arc).

As illustrated in FIG. 10, the electronic device 1 according to the present embodiment is capable of detecting rotational movement around each of three axes by the sensor 50, for example. Accordingly, the electronic device 1 according to the present embodiment may increase sensitivity of detection of the pulse wave of the subject, by combining a plurality of results detected by the sensor 50 through addition or the like. Such arithmetic operation such as addition may be performed by the controller 52, for example. In this case, the controller 52 may calculate an index of pulse wave that is based on the pulsation detected by the sensor 50.

For example, in the example illustrated in FIG. 10, a change over time in signal intensity based on the rotational movement of the sensor 50 around the α-axis and around the β-axis includes significant peaks based on the pulse wave of the subject. Accordingly, by adding up the detection results for the α-axis, the β-axis, and the γ-axis, for example, the controller 52 may increase detection accuracy for the pulse wave of the subject. Therefore, with the electronic device 1 according to the present embodiment, usefulness in measuring the pulse wave of a subject may be increased.

In an embodiment, the controller 52 of the electronic device 1 may calculate an index of the pulse wave that is based on the pulsation detected by the sensor 50. In this case, the controller 52 may combine (by addition, for example) results detected by the sensor 50 for rotational movement around at least two axes (for example, rotational movement around three axes). With the electronic device 1 according to the present embodiment, pulse wave signals for a plurality of directions may be detected. Accordingly, with the electronic device 1 according to the present embodiment, signal intensity is increased by combining detection results for a plurality of axes, compared with a case of a detection result for one axis. Accordingly, with the electronic device 1 according to the present embodiment, a signal with a good SN ratio may be detected and the detection sensitivity may be increased, and measurement may thus be stably performed.

Furthermore, in relation to a detection result for the γ-axis illustrated in FIG. 10, peaks based on the pulse wave of the subject are possibly not as significant as those in the detection results for the α-axis and the β-axis. If a detection result with a low signal level, such as the detection result for the γ-axis, is added up with the detection results for other axes, the SN ratio is possibly reduced. Furthermore, a detection result with a low signal level may sometimes be assumed to be mostly composed of noise components. In such a case, the detection result with a low signal level possibly does not include desirable pulse wave components. Accordingly, in the present embodiment, in the case where there is a detection result for an axis that does not satisfy a predetermined threshold, among detection results for a plurality of axes, the controller 52 does not have to add the detection result of such an axis.

For example, a case is assumed where the pulsation of a subject is detected by the sensor 50 in the form of rotational movement around each of the α-axis, the β-axis, and the γ-axis. Peak values in the detection results for the α-axis, the β-axis, and the γ-axis obtained as a result are assumed to exceed the predetermined threshold. In such a case, the controller 52 may add up all of the detection result for the α-axis, the detection result for the β-axis, and the detection result for the γ-axis to calculate an index of the pulse wave that is based on the pulsation detected by the sensor 50.

On the other hand, for example, it is assumed that peak values in the detection results for the α-axis and the β-axis obtained as a result of detecting the pulsation of a subject exceed the predetermined value, but a peak in the detection result for the γ-axis does not exceed the predetermined threshold. In such a case, the controller 52 may add up only the detection result for the α-axis and the detection result for the β-axis to calculate an index of the pulse wave that is based on the pulsation detected by the sensor 50.

In the case of performing such a process, the controller 52 may set, as a threshold to be used as a criterion for whether or not a detection result for an axis is to be added, different thresholds for respective axes or a same threshold for the axes. In either case, a threshold that allows the pulsation of the subject to be appropriately detected from the detection result for each axis may be set as appropriate.

In this manner, with the electronic device 1 according to the present embodiment, the controller 52 may combine only results including components at or exceeding a predetermined threshold, among results detected by the sensor 50 in the form of rotational movement around at least two axes. Accordingly, with the electronic device 1 according to the present embodiment, reduction in the SN ratio of the detection result may be suppressed. Accordingly, with the electronic device 1 according to the present embodiment, usefulness in measuring the pulse wave of a subject may be increased.

Furthermore, at the time of adding up the detection results for a plurality of axes in the manner described above, simply adding up the detection results for the axes as they are possibly results in inconvenience. This is assumed to be due to polarities of the results detected by the sensor 50 not matching each other because of positional relationships between directions of the pulsation of the subject and the sensor 50. For example, a polarity of a detection result for an axis is assumed to be reversed between a case where the pulsation is detected from the right hand of the subject by the sensor 50 and a case where the pulsation is detected from the left hand.

For example, it is assumed that, when detecting pulsation of a subject, upward peaks are approximately periodically detected in a detection result for an axis. However, it is also assumed at the same time that downward peaks are approximately periodically detected in a detection result for another axis. In the case where the polarities are reversed between detection results for a plurality of axes in the above manner, if addition is simply performed, peaks may cancel each other out, and a desirable result may not be obtained.

Accordingly, in the present embodiment, in the case where the polarities are reversed between detection results for a plurality of axes, the controller 52 may reverse the polarity of a detection result for at least one axis and then perform addition with the detection result for another axis. For example, in the case where the polarities are reversed between detection results for two axes, the controller 52 may reverse the polarity of the detection result for one axis to match that of the other axis.

In this manner, with the electronic device 1 according to the present embodiment, the controller 52 may combine the results detected by the sensor 50 in the form of rotational movement around at least two axes after aligning the polarities. With the electronic device 1 according to the present embodiment, the detection accuracy for the pulse wave of a subject may be increased. Accordingly, with the electronic device 1 according to the present embodiment, usefulness in measuring the pulse wave of a subject may be increased.

In the case of performing a process of aligning the polarities of detection results for a plurality of axes by reversing the polarity of a detection result for at least one axis, as described above, a direction of the polarity has to be determined for each detection result. Determination of the direction of the polarity may be performed by various methods. For example, the controller 52 may determine whether the peak in the detection result for each axis faces a positive direction or a negative direction in relation to signal intensity. Furthermore, for example, the controller 52 may determine whether the peak in the detection result for each axis is greater or smaller than an average value of the signal. Moreover, in the case of reversing the polarity of the detection result for at least one axis, the controller 52 may multiply the detection result for which the polarity is to be reversed by minus one.

Furthermore, the controller 52 may add or subtract a predetermined value to/from the whole of the detection result after reversing the polarity of the detection result as appropriate in the manner described above, and then, perform addition with the detection result for another axis. Moreover, before adding up the detection results for a plurality of axes, the controller 52 may correct the detection result for each axis as appropriate by, for example, performing weighting or the like on the detection result for each axis as appropriate.

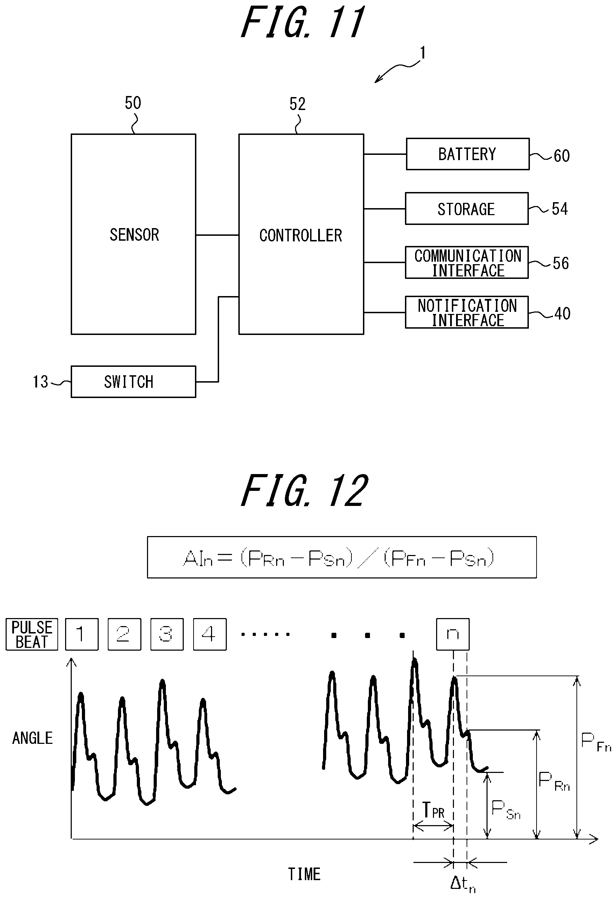

FIG. 11 is a functional block diagram illustrating a schematic configuration of the electronic device 1. The electronic device 1 illustrated in FIG. 11 includes the notification interface 40, the switch 13, the sensor 50, the controller 52, the storage 54, the communication interface 56, and the battery 60. These functional units are as described above.

FIG. 12 is a diagram illustrating an example of a pulse wave that is acquired at a wrist by the electronic device 1. FIG. 12 illustrates a case where an angular velocity sensor is used as the sensor 50 for detecting the pulsation. In FIG. 12, angular velocity acquired by the angular velocity sensor is integrated over time, and a horizontal axis indicates time and a vertical axis indicates angle. A pulse wave that is acquired possibly includes noise due to movement of the body of the subject, for example, and thus, correction may be performed using a filter for removing direct current (DC) components to extract only the pulsation component.

A method of calculating an index based on a pulse wave from a pulse wave that is acquired will be described with reference to FIG. 12. Propagation of a pulse wave is a phenomenon in which beat caused by blood that is pumped from the heart is transmitted through a wall of an artery or through blood. Beat caused by blood that is pumped from the heart reaches tips of hands and feet as a forward wave, and a part thereof is returned as a reflected wave by being reflected at a bifurcation of a blood vessel or at a part where a blood vessel diameter changes, for example. The index based on a pulse wave is a pulse wave velocity PWV of the forward wave, an amplitude PR of a reflected wave of the pulse wave, a time difference Δt between the forward wave and the reflected wave of the pulse wave, or an augmentation index (AI) that is indicated by a ratio of amplitudes of the forward wave and the reflected wave of the pulse wave, for example.

The pulse wave illustrated in FIG. 12 includes n pulse beats of a user, where n is an integer of one or more. The pulse wave is a composite wave in which the forward wave that is caused by ejection of blood from the heart and the reflected wave that is caused at a blood vessel bifurcation or at a part where a blood vessel diameter changes overlap each other. In FIG. 12, an amplitude of a peak of the pulse wave in the forward wave of each pulse beat is indicated by P_{Fn}, an amplitude of a peak of the pulse wave in the reflected wave of each pulse beat is indicated by P_{Rn}, and a minimum value of the pulse wave for each pulse beat is indicated by P_{Sn}. Furthermore, in FIG. 12, an interval between peaks of pulse beats is indicated by T_{PR}.

An index based on a pulse wave is obtained by quantifying information that is obtained from the pulse wave. For example, the PWV that is one index based on the pulse wave is calculated based on a propagation time difference of the pulse wave measured at two target portions, such as an upper arm and an ankle, and a distance between the two points. Specifically, the PWV is calculated by synchronously acquiring the pulse waves in arteries at two points (for example, an upper arm and an ankle) and by dividing a distance (L) between the two points by a time difference (PTT) in the pulse waves at the two points. For example, with respect to the amplitude P_{R} of the reflected wave that is one index based on the pulse wave, the amplitude P_{Rn} of the peak of the pulse wave in the reflected wave may be calculated, or P_{Rave} that is obtained by taking an average of the n pulse beats may be calculated. For example, with respect to the time difference Δt between the forward wave and the reflected wave of the pulse wave that is one index based on the pulse wave, a time difference Δtₙ for a predetermined pulse beat may be calculated, or Δtₐᵥₑ obtained by averaging the time differences for the n pulse beats may be calculated. For example, the AI that is one index based on the pulse wave is obtained by dividing the amplitude of the reflected wave by the amplitude of the forward wave, and is represented by AIₙ = (P_{Rn} - P_{Sn})/(P_{Fn}-P_{Sn}). AIₙ is the AI for each pulse beat. For example, the AI as one index based on the pulse wave may be obtained by measuring the pulse wave for several seconds and by calculating an average value AIₐᵥₑ of the AIₙ for respective pulse beats (n is an integer between 1 and n).

The pulse wave velocity PWV, the amplitude P_{R} of the reflected wave, the time difference Δt between the forward wave and the reflected wave, and the AI change depending on stiffness of the blood vessel wall, and may thus be used to estimate hardening of the artery. For example, if the blood vessel wall is stiff, the pulse wave velocity PWV is high. For example, if the blood vessel wall is stiff, the amplitude P_{R} of the reflected wave is great. For example, if the blood vessel wall is stiff, the time difference Δt between the forward wave and the reflected wave is small. For example, if the blood vessel wall is stiff, the AI is great. Moreover, in addition to estimating hardening of the artery, the electronic device 1 may also estimate fluidity (viscosity) of blood based on the indices based on the pulse wave. Particularly, the electronic device 1 may estimate a change in the fluidity of blood from changes in the indices based on the pulse wave acquired at a same target portion of a same subject and in a period (of several days, for example) when the state of hardening of the artery is substantially unchanged. The fluidity of blood here indicates the ease with which blood flows, and for example, if the fluidity of blood is low, the pulse wave velocity PWV is small. For example, if the fluidity of blood is low, the amplitude P_{R} of the reflected wave is small. For example, if the fluidity of blood is low, the time difference Δt between the forward wave and the reflected wave is great. For example, if the fluidity of blood is low, the AI is small.

In the present embodiment, an example is described where the electronic device 1 calculates the pulse wave velocity PWV, the amplitude P_{R} of the reflected wave, the time difference Δt between the forward wave and the reflected wave, and the AI as examples of the indices based on the pulse wave, but the indices based on the pulse wave are not limited thereto. For example, the electronic device 1 may use a second systolic blood pressure as the index based on the pulse wave.

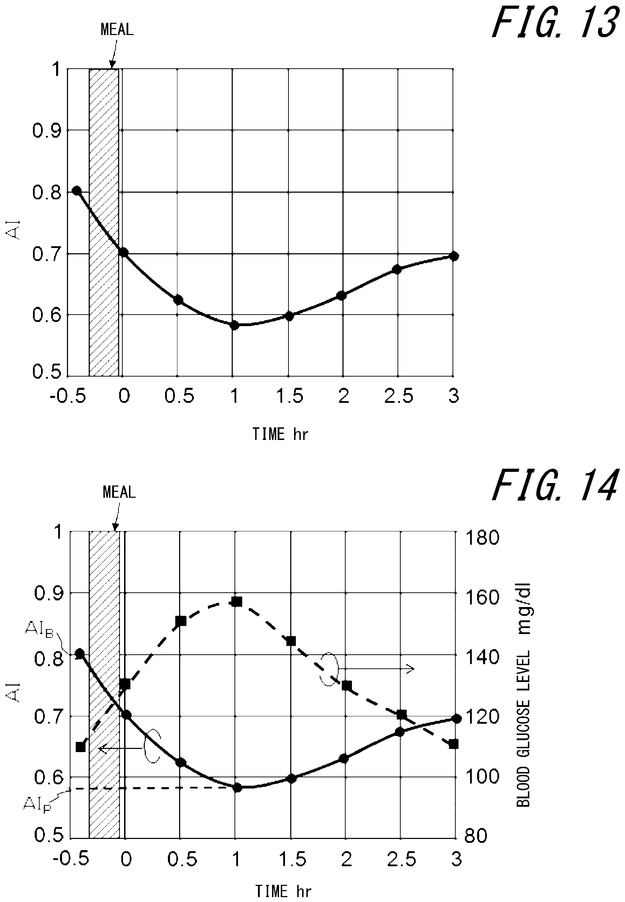

FIG. 13 is a diagram illustrating a change over time in a calculated AI. In the present embodiment, the pulse wave was acquired for about 5 seconds using the electronic device 1 including an angular velocity sensor. The controller 52 calculated the AI for each pulse beat from the acquired pulse wave, and further calculated the average value AIₐᵥₑ thereof. In the present embodiment, the electronic device 1 acquired the pulse wave at a plurality of timings before and after a meal, and calculated the average value of AI (hereinafter referred to as "AI") as an example of the index based on the acquired pulse wave. The horizontal axis in FIG. 13 indicates passing of time with a first measurement time after the meal as 0. The vertical axis in FIG. 13 indicates the AI calculated from the pulse wave acquired at a corresponding time. The subject was at rest, and the pulse wave was acquired above a radial artery.

The electronic device 1 acquired the pulse wave before a meal, immediately after the meal, and every 30 minutes after the meal, and calculated a plurality of AIs based on the respective pulse waves. The AI calculated from the pulse wave acquired before the meal was about 0.8. The AI immediately after the meal was smaller than before the meal, and the AI reached a smallest extreme value about 1 hour after the meal. The AI gradually increased until measurement ended 3 hours after the meal.

The electronic device 1 can estimate a change in fluidity of blood from a change in the calculated AIs. For example, when red blood cells, white blood cells and platelets in blood are clustered into a mass, or when viscous force is increased, the fluidity of blood is reduced. For example, when content of plasma in blood is reduced, the fluidity of blood is reduced. Such changes in the fluidity of blood are caused depending on states of glucose and lipid described later or the state of health of the subject, such as heatstroke, dehydration, hypothermia or the like. By using the electronic device 1 of the present embodiment, a subject is enabled to grasp a change in the fluidity of his/her blood before the state of health of the subject reaches a critical level. It can be estimated from the change in the AI before and after the meal illustrated in FIG. 13 that the fluidity of blood was reduced after the meal and became lowest about 1 hour after the meal, and that the fluidity of blood was gradually increased thereafter. The electronic device 1 may perform notification by using an expression "sticky" for a state where the fluidity of blood is low, and an expression "smooth" for a state where the fluidity of blood is high. For example, the electronic device 1 may determine "sticky" or "smooth" by referring to an average value of AI for the actual age of the subject. If the calculated AI is greater than the average value, the electronic device 1 may determine "smooth", and if the calculated AI is smaller than the average value, the electronic device 1 may determine "sticky". For example, the electronic device 1 may determine "sticky" or "smooth" by referring to the AI before the meal. The electronic device 1 may determine the degree of being "sticky" by comparing the AI after the meal with the AI before the meal. For example, the electronic device 1 may use the AI before the meal, or in other words, the AI when the subject is hungry, as an index of a blood vessel age (stiffness of blood vessel) of the subject. By calculating an amount of change in the calculated AI by referring to the AI for the subject before the meal, or in other words, the AI when the subject is hungry, the electronic device 1 may make small an estimation error due to the blood vessel age (stiffness of blood vessel) of the subject, and a change in the fluidity of blood may be more accurately estimated.

FIG. 14 is a diagram illustrating the calculated AI and a measurement result of a blood glucose level. The method of acquiring the pulse wave and the method of calculating the AI are the same as in the embodiment illustrated in FIG. 13. The right vertical axis in FIG. 14 indicates the blood glucose level in blood, and the left vertical axis indicates the calculated AI. The solid line in FIG. 14 indicates the AI that is calculated from the acquired pulse wave, and the dotted line indicates the measured blood glucose level. The blood glucose level was measured immediately after the pulse wave was acquired. The blood glucose level was measured using a blood glucose meter "Medisafe FIT", a product of Terumo Corporation. Compared with the blood glucose level before the meal, the blood glucose level immediately after the meal was increased by about 20 mg/dl. The blood glucose level reached a largest extreme value about 1 hour after the meal. Then, the blood glucose level gradually decreased until the end of the measurement, and reached approximately the same level as the blood glucose level before the meal about 3 hours after the meal.

As illustrated in FIG. 14, there is a negative correlation between the blood glucose levels before and after the meal and the AI calculated from the pulse wave. When the blood glucose level is increased, the red blood cells and the platelets are clustered into a mass or the viscous force is increased due to glucose in the blood, and as a result, the fluidity of the blood is possibly reduced. When the fluidity of blood is reduced, the pulse wave velocity PWV may be reduced. When the pulse-wave velocity is reduced, the time difference Δt between the forward wave and the reflected wave may be increased. When the time difference Δt between the forward wave and the reflected wave is increased, the amplitude P_{R} of the reflected wave may become small relative to the amplitude P_{F} of the forward wave. When the amplitude P_{R} of the reflected wave becomes small relative to the amplitude P_{F} of the forward wave, the AI may be reduced. The AI within several hours (3 hours in the present embodiment) after the meal is correlated with the blood glucose level, and thus, fluctuation in the blood glucose level of the subject may be estimated based on fluctuation in the AI. Furthermore, by measuring the blood glucose level of the subject and acquiring the correlation to the AI in advance, the electronic device 1 may estimate the blood glucose level of the subject from the calculated AI.

The electronic device 1 can estimate the state of glucose metabolism of the subject based on an occurrence time of AI_{P} that is the smallest extreme value of the AI detected first after the meal. As the state of glucose metabolism, the electronic device 1 estimates the blood glucose level, for example. As an example of estimation of the state of glucose metabolism, the electronic device 1 may estimate glucose metabolism abnormality for the subject (that is, the subject is estimated to be a diabetes patient), in a case where the smallest extreme value of the AI, that is, AI_{P}, that is detected first after the meal is detected after a predetermined period or longer (such as 1.5 or more hours after the meal), for example.

The electronic device 1 can estimate the state of glucose metabolism of the subject based on a difference (AI_{B} - AI_{P}) between AI_{B}, that is the AI before the meal, and the AI_{P}, that is the smallest extreme value of the AI that is detected first after the meal. As an example of estimation of the state of glucose metabolism, in the case where the (AI_{B} - AI_{P}) takes a predetermined numerical value or greater (such as 0.5 or more), a glucose metabolism abnormality may be estimated for the subject (that is, the subject is estimated to be a postprandial hyperglycemia patient), for example.

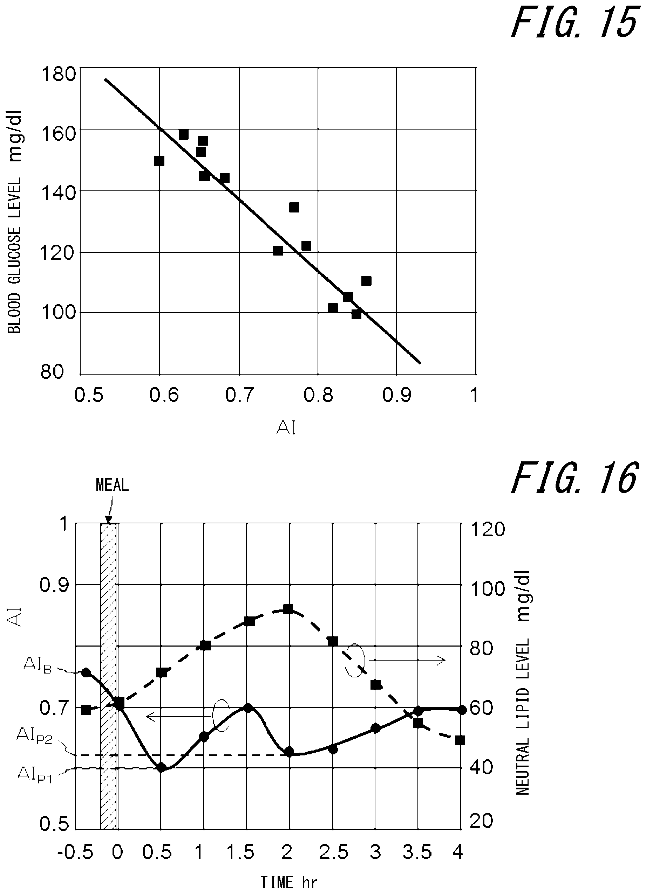

FIG. 15 is a diagram illustrating a relationship between the calculated AI and the blood glucose level. The calculated AI and the blood glucose level were acquired within one hour after the meal when fluctuation in the blood glucose level is great. Data in FIG. 15 includes pieces of data of the same subject, the pieces of data being obtained after several different meals. As illustrated in FIG. 15, there is a negative correlation between the calculated AI and the blood glucose level. The correlation is extremely high with a correlation coefficient between the calculated AI and the blood glucose level being 0.9 or more. For example, if a correlation between the calculated AI and the blood glucose level as illustrated in FIG. 15 is acquired in advance for each subject, the electronic device 1 may estimate the blood glucose level of a subject from the calculated AI.

FIG. 16 is a diagram illustrating a calculated AI and a measurement result of a neutral lipid level. The method of acquiring the pulse wave and the method of calculating the AI are the same as in the embodiment illustrated in FIG. 13. The right vertical axis in FIG. 16 indicates the neutral lipid level in blood, and the left vertical axis indicates the AI. The solid line in FIG. 16 indicates the AI that is calculated from the acquired pulse wave, and the dotted line indicates the measured neutral lipid level. The neutral lipid level was measured immediately after the pulse wave was acquired. The neutral lipid level was measured using a lipid meter "POCket LIPID", a product of Techno Medica Co., Ltd. Compared with the neutral lipid level before the meal, a largest extreme value of the neutral lipid level after the meal was increased by about 30 mg/dl. The neutral lipid level reached the largest extreme value about 2 hours after the meal. Then, the neutral lipid level gradually decreased until the end of the measurement, and reached approximately the same level as the neutral lipid level before the meal about 3.5 hours after the meal.

With respect to the smallest extreme value of the calculated AI, a first smallest extreme value AI_{P1} was detected about 30 minutes after the meal, and a second smallest extreme value AI_{P2} was detected about 2 hours after the meal. It can be estimated that the first smallest extreme value AI_{P1} detected about 30 minutes after the meal results from the blood glucose level after the meal described above. An occurrence time of the second smallest extreme value AI_{P2} detected about 2 hours after the meal approximately coincides with that of a largest extreme value of neutral lipid that was detected about 2 hours after the meal. Accordingly, it can be estimated that the second smallest extreme value AI_{P2} detected a predetermined period or longer after the meal results from the neutral lipid. As in the case of the blood glucose level, it can be seen that the neutral lipid levels before and after the meal have a negative correlation to the AI calculated from the pulse wave. Particularly, because the smallest extreme value of the AI detected a predetermined period or longer after the meal (in the present embodiment, after about 1.5 or more hours), that is, the AI_{P2}, is correlated with the neutral lipid level, fluctuation in the neutral lipid level of the subject may be estimated based on fluctuation in the AI. Furthermore, by measuring the neutral lipid level of the subject and acquiring the correlation to the AI in advance, the electronic device 1 may estimate the neutral lipid level of the subject from the calculated AI.

The electronic device 1 can estimate the state of lipid metabolism of the subject based on the occurrence time of the second smallest extreme value AI_{P2} that is detected a predetermined period or longer after the meal. As the state of lipid metabolism, the electronic device 1 estimates a lipid level, for example. As an example of estimation of the state of lipid metabolism, the electronic device 1 may estimate a lipid metabolism abnormality for the subject (that is, the subject is estimated to be a hyperlipidemia patient), in a case where the second smallest extreme value AI_{P2} is detected a predetermined period or longer after the meal (such as 4 or more hours later), for example.

The electronic device 1 can estimate the state of lipid metabolism of the subject based on a difference (AI_{B} - AI_{P2}) between AI_{B}, that is the AI before the meal, and the second smallest extreme value AI_{P2} that is detected a predetermined period or longer after the meal. As an example of estimation of a lipid metabolism abnormality, in the case where the (AI_{B} - AI_{P2}) is 0.5 or more, the electronic device 1 may estimate that the subject has a lipid metabolism abnormality (that is, the subject is estimated to be a postprandial hyperlipidemia patient), for example.

Furthermore, the electronic device 1 of the present embodiment can estimate, from the measurement results illustrated in FIGS. 14 to 16, the state of glucose metabolism of the subject, based on the first smallest extreme value AI_{P1} that is detected earliest after the meal and an occurrence time thereof. Moreover, the electronic device 1 of the present embodiment can estimate the state of lipid metabolism of the subject based on the second smallest extreme value AI_{P2} that is detected a predetermined period or longer after the first smallest extreme value AI_{P1} and the occurrence time thereof.

In the present embodiment, a case of neutral lipid is described in relation to the example of estimation of lipid metabolism, but estimation of lipid metabolism is not limited to be performed in relation to neutral lipid. For example, lipid levels estimated by the electronic device 1 include total cholesterol, high-density lipoprotein (HDL) cholesterol, low-density lipoprotein (LDL) cholesterol, and the like. Similar tendencies are observed for such lipid levels as for neutral lipid described above.

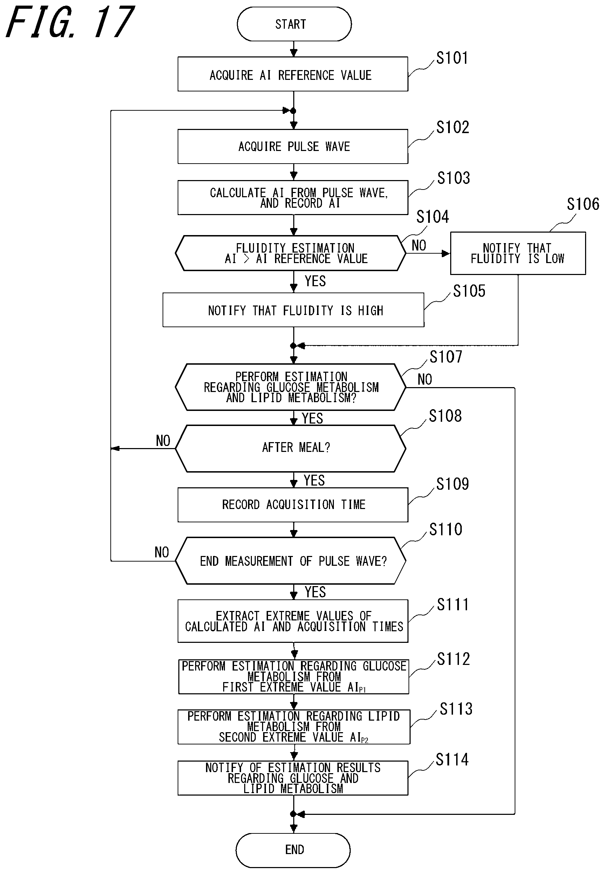

FIG. 17 is a flow illustrating a procedure for estimating, based on the AI, fluidity of blood and states of glucose metabolism and lipid metabolism. A flow of estimating fluidity of blood and states of glucose metabolism and lipid metabolism by the electronic device 1 according to the present embodiment will be described with reference to FIG. 17.

As illustrated in FIG. 17, the electronic device 1 acquires, as initial setting, an AI reference value for a subject (step S 101). As the AI reference value, an average AI that is estimated based on the age of the subject, or an AI that was acquired in advance when the subject was hungry may be used. Alternatively, the electronic device 1 may take, as the AI reference value, the AI that is determined in steps S102 to S108 to be the AI before meal, or may take, as the AI reference value, the AI that is calculated immediately before measurement of the pulse wave. In this case, the electronic device 1 performs step S101 after steps S102 to S108.

Subsequently, the electronic device 1 acquires the pulse wave (step S102). For example, the electronic device 1 determines whether a pulse wave acquired over a predetermined measurement time (such as 5 seconds) has an amplitude that is at or greater than a predetermined level. In the case where the acquired pulse wave has an amplitude that is at or greater than the predetermined level, step S103 is next performed. In the case where the amplitude is smaller than the predetermined level, step S102 is repeated (such steps are not illustrated). When a pulse wave having an amplitude that is at or greater than the predetermined level is detected in step S102, the electronic device 1 automatically acquires the pulse wave, for example.

The electronic device 1 calculates the AI from the pulse wave acquired in step S102, as the index based on the pulse wave, and stores the same in the storage 54 (step S103). The electronic device 1 may calculate, as the AI, an average value AIₐᵥₑ from AIn (n is an integer between 1 and n) for each of a predetermined number of pulse beats (such as 3 pulse beats). Alternatively, the electronic device 1 may calculate the AI for a specific pulse beat.

The AI may be calculated by performing correction based on a pulse rate P_{R}, a pulse pressure (P_{F} - P_{S}), a body temperature, a temperature at a detection portion, and the like, for example. Both the pulse rate and the pulse pressure are known to have a negative correlation to the AI, and the temperature is known to have a positive correlation to the AI. For example, in the case of performing correction, the electronic device 1 calculates, in step S103, the pulse rate and the pulse pressure together with the AI. For example, a temperature sensor may be provided in the sensor 50, and the electronic device 1 may acquire the temperature at the detection portion at the time of acquiring the pulse wave in step S102. The AI is corrected by substituting the acquired pulse rate, pulse pressure, temperature or the like in a correction formula that is created in advance.

Subsequently, the electronic device 1 compares the AI reference value acquired in step S101 and the AI calculated in step S103, and estimates fluidity of blood of the subject (step S104). In the case where the calculated AI is greater than the AI reference value (case of YES), the fluidity of blood is estimated to be high, and the electronic device 1 issues a notification indicating that the fluidity of blood is high (step S105), for example. In the case where the calculated AI is not greater than the AI reference value (case of NO), the fluidity of blood is estimated to be low, and the electronic device 1 issues a notification indicating that the fluidity of blood is low (step S106), for example.

Subsequently, the electronic device 1 checks with the subject whether to estimate the states of glucose metabolism and lipid metabolism (step S107). In the case of not performing estimation regarding the glucose metabolism and the lipid metabolism in step S107 (case of NO), the electronic device 1 ends the process. In the case of performing estimation regarding the glucose metabolism and the lipid metabolism in step S107 (case of YES), the electronic device 1 checks whether the calculated AI was acquired before the meal or after the meal (step S108). If not after the meal (that is, before meal; case of NO), step S102 is performed again, and the next pulse wave is acquired. If after the meal (case of YES), the electronic device 1 stores an acquisition time of the pulse wave corresponding to the calculated AI (step S109). In the case of subsequently acquiring the pulse wave (case of NO in step S110), step S102 is performed again, and the next pulse wave is acquired. In the case of ending measurement of pulse wave (case of YES in step S 110), step S111 and later are performed, and the electronic device 1 estimates the states of glucose metabolism and lipid metabolism of the subject.

Subsequently, the electronic device 1 extracts, from a plurality of AIs calculated in step S104, a smallest extreme value and a time thereof (step S111). For example, in the case where AIs as indicated by the solid line in FIG. 16 are calculated, the electronic device 1 extracts the first smallest extreme value AI_{P1} about 30 minutes after the meal, and the second smallest extreme value AI_{P2} about 2 hours after the meal.

Subsequently, the electronic device 1 estimates, from the first smallest extreme value AI_{P1} and the time thereof, the state of glucose metabolism of the subject (step S112). Furthermore, the electronic device 1 estimates, from the second smallest extreme value AI_{P2} and the time thereof, the state of lipid metabolism of the subject (step S113). An example of estimation of the states of glucose metabolism and lipid metabolism of the subject is the same as in the case in FIG. 16 described above, and a description thereof is omitted.

Subsequently, the electronic device 1 notifies of the estimation results of step S112 and step S113 (step S114), and ends the process illustrated in FIG. 17. For example, the notification interface 40 issues notifications such as "glucose metabolism is normal", "glucose metabolism is possibly abnormal", "lipid metabolism is normal", and "lipid metabolism is possibly abnormal". In this case, the notification interface 40 may perform notification as described above by turning on or flashing the light emitting unit, for example. Furthermore, the notification interface 40 may issue an advice such as "please see a doctor" or "please check your eating habits". The electronic device 1 then ends the process illustrated in FIG. 17.

In the present embodiment, the electronic device 1 may estimate, for a subject, the fluidity of blood and the states of glucose metabolism and lipid metabolism, based on the index based on the pulse wave. Accordingly, the electronic device 1 may estimate, for the subject, the fluidity of blood and the states of glucose metabolism and lipid metabolism in a non-invasive manner and in a short time.

In the present embodiment, the electronic device 1 may estimate the state of glucose metabolism and the state of lipid metabolism based on extreme values of the index based on the pulse wave and times thereof. Accordingly, the electronic device 1 may estimate the states of glucose metabolism and lipid metabolism of the subject in a non-invasive manner and in a short time.

In the present embodiment, for example, the electronic device 1 may estimate the states of glucose metabolism and lipid metabolism of the subject by referring to the index based on the pulse wave before a meal (when the subject is hungry). Accordingly, the fluidity of blood and the states of glucose metabolism and lipid metabolism of the subject may be accurately estimated without having to take into account the blood vessel diameter, stiffness of blood vessel and the like that do not change on a short-term basis.

In the present embodiment, by calibrating the index based on the pulse wave and the blood glucose level and the lipid level, the electronic device 1 may estimate the blood glucose level and the lipid level of the subject in a non-invasive manner and in a short time.

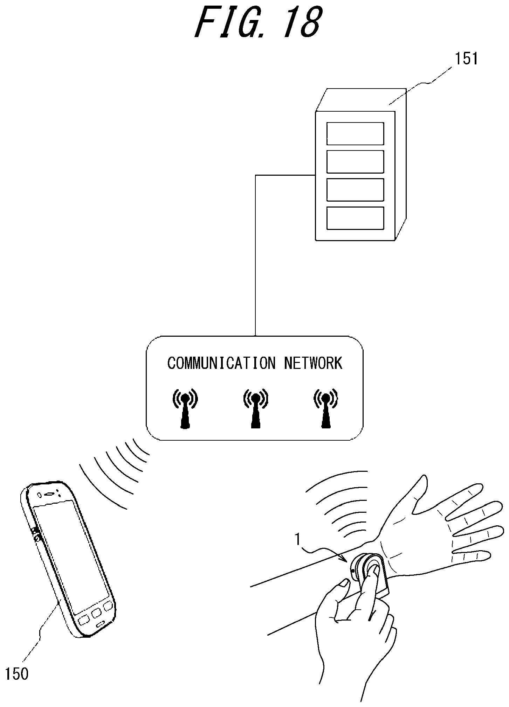

FIG. 18 is a schematic diagram illustrating a schematic configuration of a system according to an embodiment. The system illustrated in FIG. 18 includes the electronic device 1, a server 151, a mobile terminal 150, and a communication network. As illustrated in FIG. 18, the index based on the pulse wave that is calculated by the electronic device 1 is transmitted to the server 151 over the communication network, and is saved in the server 151 as personal information of the subject. The server 151 estimates the fluidity of blood and the states of glucose metabolism and lipid metabolism for the subject, by performing comparison with information about the subject acquired in the past and/or various databases. The server 151 further creates an advice best for the subject. The server 151 returns the estimation result and the advice to the mobile terminal 150 possessed by the subject. The mobile terminal 150 may construct a system that notifies of the received estimation result and advice from a display unit of the mobile terminal 150. Information from a plurality of users may be collected in the server 151 by using a communication function of the electronic device 1, and the accuracy of estimation may be further increased. Furthermore, because the mobile terminal 150 is used as notification means, the electronic device 1 does not need the notification interface 40 and may be further reduced in size. Furthermore, because estimation of the fluidity of blood and the states of glucose metabolism and lipid metabolism of the subject is performed by the server 151, calculation load on the controller 52 of the electronic device 1 may be reduced. Moreover, information about the subject acquired in the past may be saved in the server 151, and thus, load on the storage 54 of the electronic device 1 may be reduced. Accordingly, the electronic device 1 may be further reduced in size and may be simplified. Moreover, a processing speed of arithmetic operation may be increased.

In the system according to the present embodiment, the electronic device 1 and the mobile terminal 150 are connected over the communication network via the server 151, but the system according to the disclosure is not limited to such a structure. The electronic device 1 and the mobile terminal 150 may be directly connected over the communication network without using the server 151.

Characteristic examples have been described to fully and clearly disclose the present disclosure. However, the appended claims are not limited to the embodiment described above, and are to be construed as encompassing all possible modifications and alternate configurations that a person of ordinary skill in the art can make within the scope of the fundamental features indicated in the present specification.

For example, the above-described embodiment describes a case where an angular velocity sensor is included as the sensor 50, but the electronic device 1 is not limited to such a mode. The sensor 50 may include an optical pulse wave sensor including a light emitting unit and a light receiving unit, or may include a pressure sensor. Moreover, the target portion where the electronic device 1 measures the biological information is not limited to the wrist of the subject. It suffices if the sensor 50 is placed above the artery in the neck, the ankle, the thigh, the ear or the like.

For example, in the embodiment described above, the states of glucose metabolism and lipid metabolism of a subject are estimated based on the first extreme value and the second extreme value of the index based on the pulse wave and the times thereof, but the process to be performed by the electronic device 1 is not limited thereto. There may be a case where only one extreme value appears or a case where no extreme values appear, and the electronic device 1 may estimate the states of glucose metabolism and lipid metabolism of a subject based on an overall tendency (such as an integral value, Fourier transform or the like) of the change over time in the calculated index based on the pulse wave. Furthermore, the electronic device 1 may estimate the states of glucose metabolism and lipid metabolism of a subject based on a time range when the index based on the pulse wave is at or below a predetermined value, instead of by extracting extreme values of the index based on the pulse wave.

For example, in the embodiment described above, a case of estimating the fluidity of blood before and after a meal is described, but the process to be performed by the electronic device 1 is not limited thereto. The electronic device 1 may estimate the fluidity of blood before, after and during exercise, or may estimate the fluidity of blood before, after and during a bath.

In the embodiment described above, the electronic device 1 is described to measure the pulse wave, but the pulse wave does not necessarily have to be measured by the electronic device 1. For example, the electronic device 1 may be connected to an information processing apparatus, such as a computer or a mobile phone, in a wired or wireless manner, and may transmit information about the angular velocity acquired by the sensor 50 to the information processing apparatus. In this case, the information processing apparatus may measure the pulse wave based on the information about the angular velocity. The information processing apparatus may also perform the process of estimating the glucose metabolism and the lipid metabolism. In the case where the information processing apparatus connected to the electronic device 1 is to perform various types of information processing, the electronic device 1 does not have to include the controller 52, the storage 54, the notification interface 40, and the like. Furthermore, in the case where the electronic device 1 is connected to the information processing apparatus in a wired manner, the electronic device 1 does not have to include the battery 60 and power may be supplied from the information processing apparatus.

Moreover, in an embodiment, the housing 10 and the stand part 20 of the electronic device 1 may have shapes other than those illustrated in FIGS. 3 to 6. For example, in an embodiment, the housing 10 of the electronic device 1 may have a shape of a disc or a triangle. Furthermore, the stand part 20 of the electronic device 1 may have any shape as long as the stand part 20 can be placed on an upper surface of a top board (a table top) of a supporting structure such as a table or a desk. In an embodiment, the electronic device 1 may have various structures including a housing, at least a part of which includes the sensor 50, and the stand part 20 that supports the housing 10 and that leans against the target portion via the housing 10.

Moreover, the controller 52 of the electronic device 1 may estimate, from the index of the pulse wave, at least one of the glucose and lipid metabolism, the blood glucose level, and the lipid level. Furthermore, the electronic device 1 may function as a diet monitor that monitors progress of a diet of the subject, or as a blood glucose meter that monitors the blood glucose level of the subject.

### REFERENCE SIGNS LIST

- 1: electronic device
- 10: housing
- 11: first contact part
- 12: second contact part
- 13: switch
- 14: protruding part
- 20: stand part
- 22: press part
- 24: extendable part
- 26: receiving part
- 30: substrate
- 40: notification interface
- 50: sensor
- 52: controller
- 54: storage
- 56: communication interface
- 60: battery
- 70: elastic member
- 150: mobile terminal
- 151: server

## Claims

1. An electronic device comprising:
a sensor capable of detecting pulsation at a target portion of a subject;
a housing, at least a part of which includes the sensor;
a stand part that supports the housing, and that leans against the target portion via the housing; and
an elastic member disposed between the housing and the stand part.

2. The electronic device according to claim 1, comprising a stopper that allows the housing to partially come into contact with the stand part when a position of the housing is displaced relative to the stand part by deformation of the elastic member.

3. The electronic device according to claim 2, wherein
the stopper includes a protruding part formed on one of the housing and the stand part, and a receiving part formed on other one of the housing and the stand part, and
the receiving part is formed to be capable of receiving the protruding part.

4. The electronic device according to any one of claims 1 to 3, wherein the stand part is formed to be capable of extending or contracting in a predetermined direction in a stepwise manner.

5. The electronic device according to claim 4, wherein the stand part is formed to be capable of extending or contracting in the predetermined direction, so that a position of the housing in a height direction is made adjustable.

6. The electronic device according to claims 1 to 5, wherein the housing includes a first contact part for contacting the target portion, and a second contact part for contacting a periphery of a position where the first contact part contacts the target portion.

7. The electronic device according to claim 6, wherein the first contact part protrudes farther from the housing than the second contact part does.

8. The electronic device according to any one of claims 1 to 7, wherein the elastic member is capable of deforming in response to the pulsation at the target portion.

9. The electronic device according to any one of claims 1 to 8, wherein the elastic member is three-dimensionally deformable.

10. The electronic device according to any one of claims 1 to 9, wherein the elastic member elastically deforms to an extent that allows the sensor to detect the pulsation at the target portion.

11. The electronic device according to any one of claims 1 to 10, wherein the sensor detects the pulsation at the target portion as a part of rotational movement around a predetermined axis.

12. The electronic device according to claim 11, wherein the sensor detects the pulsation at the target portion as rotational movement around at least two axes.

13. The electronic device according to claim 12, wherein the sensor detects the pulsation at the target portion as rotational movement around three axes.

14. The electronic device according to any one of claims 1 to 13, wherein the sensor is a gyro sensor.

15. The electronic device according to any one of claims 1 to 14, further comprising a controller for calculating an index, of a pulse wave, that is based on the pulsation detected by the sensor, wherein
the controller combines results detected by the sensor as rotational movement around at least two axes.

16. The electronic device according to claim 15, wherein the controller combines only results that each include a component at or exceeding a predetermined threshold, among the results detected by the sensor as rotational movement around at least two axes.

17. The electronic device according to claim 15 or 16, wherein the controller combines the results detected by the sensor as rotational movement around at least two axes after aligning polarities.

18. The electronic device according to any one of claims 1 to 17, wherein the elastic member is an elastic member that is deformable along at least one axis among three mutually perpendicular axes.

19. The electronic device according to any one of claims 15 to 18, wherein the controller estimates, from the index of the pulse wave, at least one of glucose and lipid metabolism, a blood glucose level, and a lipid level.

20. The electronic device according to any one of claims 1 to 19, wherein the electronic device functions as a diet monitor that monitors progress of a diet of the subject, or a blood glucose meter that monitors a blood glucose level of the subject.
